(19) Europäisches Patentamt
European Patent Office
Office européen des brevets

(11) Publication number: **0 017 478**
**B1**

(12) # EUROPEAN PATENT SPECIFICATION

(45) Date of publication of patent specification: **30.03.83**

(51) Int. Cl.³: **C 07 D 473/10,**
**A 61 K 31/52**

(21) Application number: **80301051.1**

(22) Date of filing: **02.04.80**

(54) **Dialkoxy- and dioxacycloalkyl-alkyltheobromines, pharmaceutical compositions containing them and their preparation.**

(30) Priority: **05.04.79 GB 7912051**

(43) Date of publication of application:
**15.10.80 Bulletin 80/21**

(45) Publication of the grant of the patent:
**30.03.83 Bulletin 83/13**

(84) Designated Contracting States:
**BE CH DE FR GB IT LI NL SE**

(56) References cited:
**DD - C - 129 911**
**DE - B - 1 212 542**
**FR - A - 1 523 273**
**FR - M - 7 390**

**The Merck Index, 9th ed., 1976, Merck & co., Inc., Rahway, N.J., U.S.A. page 6933, para. 6931**

**The file contains technical information submitted after the application was filed and not included in this specification**

(73) Proprietor: **BEECHAM - WUELFING GmbH & Co. KG**
**Stresemannallee 6 P.O. Box 25**
**D-4040 Neuss (DE)**

(72) Inventor: **Goring, Joachim Ewald**
**Sauerweinstrasse 25**
**D-3212 Gronau (Leine) (DE)**

(74) Representative: **Russell, Brian John et al,**
**European Patent Attorney Beecham Pharmaceuticals Great Burgh Yew Tree Bottom Road**
**Epsom Surrey, KT18 5XQ (GB)**

(56) References cited:
**E. Schröder, C. Rufer, R. Schmiechen, Arzneimittelchemie, vol. II, 1976, Georg Thieme Verlag, pages 65, 66**
**CHEMICAL ABSTRACTS, vol. 87, no. 1, 04-07-1977 ref. 6023c, page 491 Columbus, Ohio, US**
**CHEMICAL ABSTRACTS, vol. 88, no. 5, 30-01-1978, ref. 37836v, page 50 Columbus, Ohio, US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 017 478 B1

Dialkoxy- and dioxacycloalkyl-alkyltheobromines, pharmaceutical compositions containing them and their preparation

1-(5-Oxohexyl)theobromine(pentoxyfilline) is known as a peripheral vasodilator from NL—A—7309709.

The compound of the formula (I):

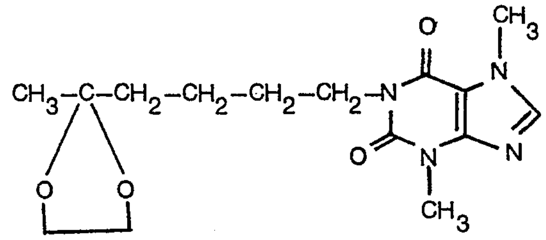

(I)

which is a ketal of pentoxyfilline, was disclosed in DD—C—129911 as being a chemical intermediate but no pharmacological activity was ascribed to it. It has now been found that the compound of the formula (I) and certain related compounds have similar pharmacological properties to pentoxyfilline and so may be used to promote blood flow through skeletal muscles which is desirable for the treatment of intermittent claudication.

The present invention provides a pharmaceutical composition containing a compound of the formula (II):

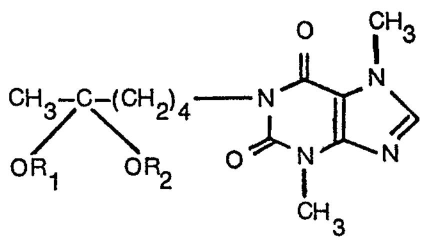

(II)

wherein $R_1$ is a $C_{1-4}$ alkyl group and $R_2$ is a $C_{1-4}$ alkyl group optionally linked to $R_1$ so that the $OR_1$ and $OR_2$ moieties and the carbon atom to which they are attached form a 1,3-dioxacyclohexa-2,2-diyl, 1,3-dioxacyclopenta-2,2-diyl or 1,3-dioxacyclohepta-2,2-diyl diradical.

Suitable $C_{1-4}$ alkyl groups include methyl, ethyl, n-propyl, iso-propyl and n-butyl groups.

Most suitably $R_1$ and $R_2$ both represent the same $C_{1-4}$ alkyl group or alternatively are linked.

A particularly suitable acyclic value for $R_1$ is the ethyl group. A particularly suitable acyclic value for $R_2$ is the ethyl group.

A preferred compound of the formula (II) is that wherein $R_1$ and $R_2$ are linked so that the $C(OR_1)(OR_2)$ moiety is a 1,3-dioxacyclopenta-2,2-diyl diradical.

The compounds of this invention may be used to treat vascular disorders in the same manner as pentoxyphilline is used. Thus for example they may be formulated into orally administrable unit dose forms that contain 100—500 mg of the compound of the formula (II). Such unit dose forms may be administered 1—4 times daily in such a manner that the daily dose for a 70 kg adult will usually be in the range 300—1500 mg per day.

Particularly suitable unit dose forms are tablets and capsules. These forms will contain excipients in normal manner, for example lubricants such as magnesium stearate, disintegrants such as microcrystalline cellulose or sodium starch glycollate.

The present invention also provides a process for the preparation of the compositions containing a compound of the formula (II), which process comprises bringing a compound of the formula (II) into association with a pharmaceutically acceptable carrier.

The compositions of this invention may be fabricated by conventional methods of blending, compressing, filling and the like, for example those methods known as suitable for pentoxyphilline.

The compounds of the formula (II) may be prepared by the method described in DDR Patentschrift 129911. Thus suitable methods comprise ketalising pentoxyfilline in conventional manner or reacting a salt of the 3,7-dimethylxanthine with $CH_3 . C(OR_1) . (CH_2)_4 . Cl$ or the like.

Ketalisation of pentoxyphilline may be brought about by reaction with a diol in benzene in the presence of p-toluene sulphonic and in conditions under which the water produced is removed. After about 1 day the benzene layer may be washed to remove excess diol and the benzene evaporated.

Acyclic ketals may be prepared by the reaction with an orthoester at ambient temperature. In this form of the process a large excess of the orthoester may be used so that the orthoester also acts as solvent. A catalyst such as Amberlyst 15 (Registered Trade Mark) may also be employed (see Patwardhan et al., Synthesis, 1974, page 340).

Purifications of the initially produced product may be effected in conventional manner, for example by crystallisation from petroleum ether.

Compounds of the formula (II) other than the compound of the formula (I) and are novel, and as such form an aspect of the present invention.

The compound of the formula (I) as a medicament for the promotion of blood flow through skeletal muscle is novel, and as such also forms an aspect of the present invention.

The following Examples illustrate the preparation of the pharmaceutical compositions and the compounds of the present invention.

Example 1
Tablet composition

2-Methyl-2-(theobrominyl-butyl-4)-1,3-dioxolane, magnesium stearate and microcrystalline cellulose may be blended together and passed through a 40 mesh sieve (UK). The mixture may be tabletted on a conventional rotary machine to produce a batch of 5000 tablets of the following composition:

| | |
|---|---|
| 1-Methyl-2-(theobrominyl-butyl-4)-1,3-dioxolane | 100 mg |
| Magnesium stearate | 0.2 mg |
| Microcrystalline cellulose | 98.8 mg |

Example 2
Capsule composition

2-Methyl-2-(theobrominyl-butyl-4)-1,3-dioxolane and magnesium stearate may be blended together and passed through a 40 mesh sieve (UK). The resulting mixture may be filled into two-part hard gelating capsules to produce a batch of 5000 of the following composition:

| | |
|---|---|
| 2-Methyl-2-(theobrominyl-butyl-4)-1,3-dioxolane | 200 mg |
| Magnesium stearate | 0.2 mg |

Demonstration of preparation

1-(5-Oxohexyl)-3,7-dimethylxanthine (10.2 g), benzene (180 ml) and 1,2-ethandiol (5.1 ml) were refluxed with p-toluenesulphonic acid (10 mg). After three hours the reaction was complete. The reaction water had been collected in a water separator. The reaction mixture was washed several times with a solution of sodium carbonate and water and then dried over sodium sulphate, filtered and evaporated to dryness under reduced pressure. The residue was recrystallized from ethyl acetate to give 2-methyl-2-(theobrominylbutyl-4)-1,3-dioxolane (5.9 g) in the form of a white powder, melting point 93°C.

The structure was confirmed by NMR spectroscopy and elemental analysis.

Illustration of pharmacological effectiveness
Methodology

Cats of either sex were anaesthetized by i.p. injection of urethane/chloralose (120/60 mg/kg). The intraduodenal (i.d.) administration of compounds was conducted by means of a plastic catheter which was inserted into the duodenum following midline incision at the abdominal cavity.

i) $pO_2$-Measurements

Measurement of muscle surface $pO_2$. The skin above the measuring site (3—4 mm in diameter) was removed and one multiwire-surface electrode (Eschweiler, Kiel) was placed on the gastrocnemius muscle of each hindlimb. The femoral artery in one hindlimb was ligated in order to induce ischaemia. Muscle temperature was controlled by means of a thermocouple (Ellab, Copenhagen). The electrode current was measured every 6 to 8 s and collected for periods of 4 min (Hewlett-Packard programmable data logger system 3051 A). After each period, mean value and standard deviation was calculated.

ii) Skeletal muscle contractility

After dissection of the skin of the calf muscles, the sciatic nerve was cut about 3 cm proximal to the knee. The tendon of the calf muscles was cut and connected with an isometric force transducer (SWEMA, SG 3). In order to maintain constant differences and a resting tension of 100 p in cats and 25 p in rats, the hindlimb was fixed at the tibia by means of a clamp. Direct stimulation of the muscles consisted of square wave pulses of 4 msec duration at a frequency of 2 Hz and at a voltage 50 V in cats. In order to keep the muscles wet and at a normal temperature, the muscles were continuously superfused with 0.9% w/v NaCl solution (38°C). Femoral blood flow was restricted by a graded occlusion of the artery leading to a reduction of contractility by ca. 30%. After having reached a constant level of the contraction force, the appropriate vehicle (NaCl or Methocel) was injected, followed by the test substance.

Results obtained with 2-methyl-2-(theobrominyl-butyl-4)-1,3-dioxolane
i) $pO_2$-Measurements

| Dosage (mg/kg) i.d.* | n | Hypoxic tissue | | | Normoxic tissue | | |
|---|---|---|---|---|---|---|---|
| | | $C_s$ | $\overline{\Delta pO_2}$ (Torr) | E (Torr) | $C_s$ | $\overline{\Delta pO_2}$ (Torr) | E (Torr) |
| 12.5 | 4 | 1 | 3.7 | 3.7 | 1 | 4.8 | 4.8 |

ii) Skeletal muscle contractility

| Dosage (mg/kg) i.d.* | n | Increase of contractility (% of initial values) |
|---|---|---|
| 12.5 | 2 | +40.0; +18.2 |

control values (Methocel°): ±0

n=number of animals
$C_s$=significance coefficient=number of measuring sites with significant $pO_2$ increase per total number of measuring sites
$\overline{\Delta pO_2}$=mean $pO_2$ increase in experiments with significant $pO_2$ increase (Torr)
E=efficiency-index=$C_s \times \overline{\Delta pO_2}$ (Torr)
Control values: E=between 0.1 and 1.2 Torr
i.d.*=intraduodenal administration of a suspension in Methocel°.

No toxic effects were observed at the above test dosages.

**Claims**

1. A pharmaceutical composition characterised by containing a compound of the formula (II):

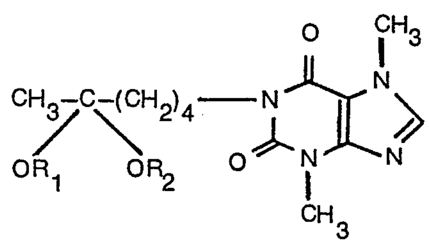

(II)

wherein $R_1$ is a $C_{1-4}$ alkyl group and $R_2$ is a $C_{1-4}$ alkyl group optionally linked to $R_1$ so that the $OR_1$ and $OR_2$ moieties and the carbon atom to which they are attached form a 1,3-dioxacyclohexa-2,2-diyl, 1,3-dioxacyclopenta-2,2-diyl or 1,3-dioxacyclohepta-2,2-diyl diradical.

2. A pharmaceutical composition according to Claim 1 characterised by containing a compound of the formula (II), wherein $R_1$ and $R_2$ are the same $C_{1-4}$ alkyl groups.

3. A pharmacetical composition according to Claim 1 characterised by containing a compound of the formula (II), wherein the $OR_1$ and $OR_2$ moieties and the carbon atom to which they are attached form a 1,3-dioxacyclohexa-2,2-diyl, 1,3-dioxacyclopenta-2,2-diyl or 1,3-dioxacyclohepta-2,2-diyl diradical.

4. A pharmaceutical composition according to Claim 3 characterised by containing 2-methyl-2-(4-theobrominylbutyl)-1,3-dioxolane.

5. A pharmaceutical composition according to any one of Claims 1 to 4 which is a tablet.

6. A pharmaceutical composition according to any one of Claims 1 to 4 which is a capsule.

7. A process for the preparation of a pharmaceutical composition according to Claim 1 characterised by bringing a compound of the formula (II) into association with a pharmaceutically acceptable carrier.

8. A process according to Claim 7, characterised by blending the compound of the formula (II) with the carrier and optionally thereafter compressing the resultant blend into a tablet or filling the resultant blend into a capsule.

9. A compound of the formula (II) as defined in Claim 1, other than 2-methyl-2-(4-theobrominyl-butyl)-1,3-dioxolane.

10. 2-Methyl-2-(4-theobrominyl-butyl)-1,3-dioxolane for the promotion of blood flow through skeletal muscle.

## Revendications

1. Composition pharmaceutique, caractérisée en ce qu'elle renferme un composé de formule (II):

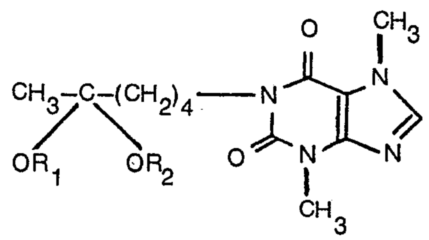

(II)

dans laquelle $R_1$ est une groupe alcoyle en $C_{1-4}$ et $R_2$ est un groupe alcoyle en $C_{1-4}$ lié facultativement à $R_1$ de sorte que les fractions molaires $OR_1$ et $OR_2$ et l'atome de carbone sur lequel elles sont fixées forment un diradical 1,3-dioxacyclohexa-2,2-diyle, 1,3-dioxacyclopenta-2,2-diyle ou 1,3-dioxacyclohepta-2,2-diyle.

2. Composition pharmaceutique suivant la revendication 1, caractérisée en ce qu'elle renferme un composé de formule (II) dans laquelle $R_1$ et $R_2$ sont les mêmes groupes alcoyle en $C_{1-4}$.

3. Composition pharmaceutique suivant la revendication 1, caractérisée en ce qu'elle renferme un composé de formule (II) dans laquelle les fractions molaires $OR_1$ et $OR_2$, et l'atome de carbone sur lequel elles sont fixées forment un diradical 1,3-dioxacyclohexa-2,2-diyle, 1,3-dioxacyclopenta-2,2-diyle ou 1,3-dioxacyclohepta-2,2-diyle.

4. Composition pharmaceutique suivant la revendication 3, caractérisée en ce qu'elle renferme du 2-méthyl-2-(4-théobrominylbutyl)-1,3-dioxolane.

5. Composition pharmaceutique suivant l'une quelconque des revendications 1 à 4, constituée par un comprimé.

6. Composition pharmaceutique suivant l'une quelconque des revendications 1 à 4, constituée par une gélule ou capsule.

7. Procédé pour la préparation d'une composition pharmaceutique suivant la revendication 1, caractérisé en ce qu'on associe un composé de formule (II) avec un véhicule ou excipient pharmaceutiquement acceptable.

8. Procédé suivant la revendication 7, caractérisé en ce qu'on mélange le composé de formule (II) avec le véhicule ou l'excipient, puis on presse ensuite facultativement le mélange résultant en un comprimé, ou bien on introduit le mélange résultant dans une gélule ou capsule.

9. Composé de formule (II) suivant la revendication 1, autre que le 2-méthyl-2-(4-théobrominylbutyl)-1,3-dioxolane.

10. 2-Méthyl-2-(4-théobrominyl-butyl)-1,3-dioxolane destiné à favoriser la circulation sanguine dans les muscles du squelette.

## Patentansprüche

1. Eine pharmazeutische Zusammensetzung, gekennzeichnet durch den Gehalt an einer Verbindung der Formel (II):

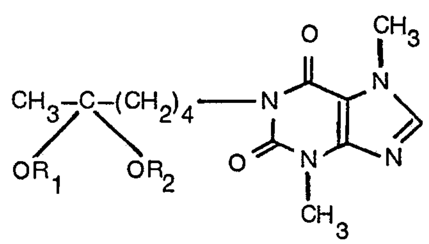

(II)

in der $R_1$ eine $C_{1-4}$-Alkylgruppe ist und $R_2$ eine $C_{1-4}$-Alkylgruppe ist, die gegebenenfalls mit $R_1$ verbunden ist, so daß die $OR_1$- und $OR_2$-Reste und das Kohlenstoffatom, an das die gebunden sind, ein 1,3-Dioxacyclohexa-2,2-diyl-, 1,3-Dioxacyclopenta-2,2-diyl- oder 1,3-Dioxacyclohepta-2,2-diyl-Diradikal bilden.

2. Eine pharmazeutische Zusammensetzung gemäß Anspruch 1, gekennzeichnet durch einen Gehalt an einer Verbindung der Formel (II), in der $R_1$ und $R_2$ die gleichen $C_{1-4}$-Alkylgruppen sind.

3. Eine pharmazeutische Zusammensetzung gemäß Anspruch 1, gekennzeichnet, durch einen Gehalt an einer Verbindung der Formel (II), in der die $OR_1$- und $OR_2$-Reste und das Kohlenstoffatom, an das sie gebunden sind, ein 1,3-Dioxacyclohexa-2,2-diyl-, 1,3-Dioxacyclopenta-2,2-diyl- oder 1,3-Dioxacyclohepta-2,2-diyl-Diradikal bilden.

4. Eine Pharmazeutische Zusammensetzung gemäß Anspruch 3, gekennzeichnet durch einen Gehalt an 2-Methyl-2-(4-theobrominyl-butyl)-1,3-dioxolan.

5. Eine pharmazeutische Zusammensetzung gemäß einem der Ansprüche 1 bis 4, die eine Tablette ist.

6. Eine pharmazeutische Zusammensetzung gemäß einem der Ansprüche 1 bis 4, die eine Kapsel ist.

7. Ein Verfahren zur Herstellung einer pharmazeutischen Zusammensetzung gemäß Anspruch 1, dadurch gekennzeichnet, daß eine Verbindung der Formel (II) mit einem pharmakologisch verträglichen Träger vereinigt wird.

8. Ein Verfahren gemäß Anspruch 7, dadurch gekennzeichnet, daß eine Verbindung der Formel (II) mit einem Träger vermischt wird und gegebenenfalls anschließend das entstandene Gemisch in eine Tablette komprimiert oder das entstandene Gemisch in eine Kapsel gefüllt wird.

9. Eine Verbindung der Formel (II), wie in Anspruch 1 definiert, verschieden von 2-Methyl-2-(4-theobrominyl-butyl)-1,3-dioxolan.

10. 2-Methyl-2-(4-theobrominyl-butyl)-1,3-dioxolan zur Förderung des Blutflusses durch Skelettmuskeln.